# EUROPEAN PATENT SPECIFICATION

(11) **EP 1 334 763 B1**
(45) Date of publication and mention of the grant of the patent: **20.12.2006**
(21) Application number: 01974897.9
(22) Date of filing: 23.10.2001
(51) Int. Cl.: B01D 69/00, A61L 31/06, A61L 31/14

(54) **HYDROPHILIZED MEMBRANE AND METHOD OF HYDROPHILIZATION THEREFOR**
HYDROPHILIERTE MEMBRAN UND HYDROPHILIERUNGSVERFAHREN DAFÜR
MEMBRANE HYDROPHILISEE ET PROCEDE D'HYDROPHILISATION DE CELLE-CI

(30) Priority: 24.10.2000 JP 2000323859
(43) Date of publication of application: 13.08.2003
(73) Proprietor: KANEKA CORPORATION, Osaka-shi, Osaka 530-8288 (JP)
(72) Inventor: KOBAYASHI, Akira, Settsu-shi, Osaka 566-0072 (JP); OIDE, Yasuhito, Takatsuki-shi, Osaka 569-0816 (JP); FUJITA, Koji, Osaka-shi, Osaka 545-0052 (JP); TANI, Nobutaka, Osaka-shi, Osaka 545-0004 (JP)
(74) Representative: Vossius & Partner
(86) International application number: PCT/JP2001/009277
(87) International publication number: WO 2002/034374

(56) References cited:
- EP-A- 0 341 151
- EP-A- 0 402 196
- EP-A2- 0 169 105
- WO-A-94/00222
- WO-A1-84/00015
- JP-A- 3 143 534
- JP-A- 61 002 743
- JP-A- 61 133 105
- JP-A- 63 141 609
- JP-A- 63 277 251
- PATENT ABSTRACTS OF JAPAN vol. 016, no. 369 (C-0972), 10 August 1992 (1992-08-10) & JP 04 118033 A (MATERIAL ENG TECH LAB INC;OTHERS: 01), 20 April 1992 (1992-04-20)
- PATENT ABSTRACTS OF JAPAN vol. 010, no. 280 (C-374), 24 September 1986 (1986-09-24) & JP 61 101209 A (ASAHI MEDICAL KK), 20 May 1986 (1986-05-20)

## Description

The present invention relates to a membrane endowed with high blood compatibility and decreased in interaction with blood cells (particularly blood platelets) by conducting hydrophilization of a hydrophobic membrane and a process for hydrophilization. More specifically, the present invention relates to a hydrophilized membrane which can suitably be used as a plasma separation membrane, a double plasma filtration membrane, a blood filtration membrane and a dialysis membrane, and a process for hydrophilization of the hydrophobic part to be in contact with blood for medical equipment in which the part to be in contact with blood is hydrophobic.

Though a hydrophobic membrane generally is advantageous in that membrane strength is high and dry storage is possible, weak points such as low filtration property, a tendency to adsorb protein and low blood compatibility have been pointed out. And so, attempts have been made from long ago for technical development regarding hydrophilization of a hydrophobic membrane. Typical methods are (1) the method of adsorbing hardly volatile water-soluble polyvalent alcohol such as glycerin to a hydrophobic membrane; (2) the method of adsorbing water-soluble polymer such as polyethylene glycol, polyvinyl pyrrolidone and polyvinyl alcohol to a hydrophobic membrane (JP-A-63-31501); (3) the method of immobilizing a hydrophilic polymer to a hydrophobic membrane, the method of chemically bonding a hydrophilic monomer such as acrylamide to the surface of a hydrophobic membrane (JP-A-2-59032); (4) the method of immobilizing a hydrophilic polymer on a membrane by crosslinking and insolubilizing the hydrophilic polymer by irradiating the membrane in a wet state (JP-A-4-300636), the method of insolubilizing and immobilizing the hydrophilic polymer to a membrane by heat treatment in a dry state (JP-A-9-103664); (5) the method of sulfonating the surface of a hydrophobic membrane (JP-A-63-54452); (6) the method of preparing a membrane from a mixture of hydrophilic polymer such as polyethylene glycol and polyvinyl pyrrolidone and hydrophobic polymer dope (JP-A-61-93801); (7) the method of introducing a hydrophilic group to the surface of a membrane by treating with an alkaline aqueous solution (for example NaOH, KOH) (JP-A-58-93734); (8) the method of treating a hydrophobic porous membrane with an aqueous solution of water-soluble polymer after impregnating in alcohol and then insolubilizing by heat treatment or radiation after drying (JP-B-54-17978).

Of these, the above methods (1) to (3) have been known to one skilled in the art from long ago as a usual method for hydrophilizing a hydrophobic membrane, but as can easily be predicted, have the weakness of the hydrophilic properties being lost, as the hydrophilizing agent used in each of the methods is detached from the hydrophobic membrane after coming into contact with water once. Also, according to the usage, mixing a hydrophilizing agent in the filtrate is not desired in some cases. As an improved method of the above method (2), the method of making the hydrophilizing agent difficult to detach from the membrane by making the hydrophilizing agent difficult to dissolve in water by further applying radiation or conducting heat treatment after conducting method (2), has been suggested. However, there are problems such as low membrane strength and effects which are not yet of a sufficiently satisfactory degree.

Also, the above methods (4) and (5) are advantageous in that the hydrophilic properties of the hyrdophobic membrane are maintained almost permanently and the hydrophilizing agent does not elute into the filtrate, but are weak in that the treatment process is relatively complicated and uneconomical.

The above method (6) has also been known for a long time, but has problems such as difficulty in controlling the remnant state of the hydrophilic polymer within the hydrophobic membrane, change in filtration properties over time and gradual elution of the hydrophilic polymer. Also, regarding method (7), there are problems such as limitation of the treated material and a decrease in membrane strength due to the alkaline aqueous solution treatment. Regarding method (8), there are problems such as a decrease in membrane strength due to drying, heat treatment or radiation exposure when insolubilizing.

In this way, in the above conventional arts, the hydrophilizing agent usually elutes into the filtrate. In order to prevent this, complicated and uneconomical treatment was employed and obtaining an excellent hydrophilized membrane was difficult. Furthermore, improving water permeability by hydrophilization is the main object of these arts and only few mention interaction with blood (particularly blood cells). Examples of a method for hydrophilizing the membrane and also imparting high blood compatibility are the method of coating with a polysaccharide having an anticoagulant effect such as heparin and the method of chemically immobilizing polyethylene glycol by covalent bonding, but both are complicated and have insufficient effects. In addition, a sufficiently satisfactory method in terms of safety and cost has not yet obtained. Presently, a hydrophilization method and hydrophilized membrane which does not trigger deterioration of the membrane material or decrease in strength which accompanies hydrophilization, has high blood compatibility and safety and is simple and economical, has not been obtained.

EP-A-0 341 151 discloses a hydrophilic porous membrane obtained by covering a porous membrane substrate with a non-ionic, amino-acid-based or non-ionic fluorine-based surface active agent. For the covering, the porous membrane substrate is immersed in a solution containing the surface active agent and then drying the membrane substrate. A different hydrophilic porous membrane is obtained by providing the surfaces of a porous membrane substrate and inner pore surfaces with a polar group and then covering the membrane substrate with a hydrophilic polymer film. For this covering, after introduction of the polar group to the surfaces of the porous membrane substrate and inner pore surfaces, the membrane substrate is immersed in a solution containing a hydrophilic substance and then dried. In a liquid filter using any of these hydrophilic porous membranes, the porous membrane is disposed in a housing such as to divide the housing inner space into two sub-spaces respectively communicating with a liquid inlet and a filtrate outlet provided on the housing.

WO-A-94/00222 describes asymmetrical microporous hollow fibers incorporating a low molecular weight surfactant and the process for the production of the hollow fibers. The hollow fibers are said to have significantly improved flux and rewetting characteristics. The process involves passing a polymeric solution through an outer annulus of a die to create an annular stream and a precipitating fluid through the inner orifice of the die creating a stream within the annular stream resulting in hollow fiber formation. The process further involves incorporating a low molecular weight surfactant into the hollow fibers at any one of several stages during the process.

As a result of dedicated research regarding interaction between a membrane obtained by hydrophilization of a hydrophobic membrane and blood cells (particularly blood platelets) and a method for immobilizing the hydrophilizing agent to the membrane, it was found that after bringing a substance having surface activity with a number average molecular weight of 500 to 8,000 (hydrophilizing agent) into contact with a hydrophobic membrane, by thoroughly rinsing with a solvent into which the hydrophilizing agent is dissolved or can be dissolved, detaching the elutable hydrophilizing agent and practically adsorbing an extremely small amount of the hydrophilizing agent to the membrane without conducting immobilizing treatment such as drying, heating, electron irradiation or crosslinking, elution of the hydrophilizing agent does not occur, interaction with blood cells (particularly blood platelets) becomes surprisingly low and high blood compatibility can be imparted to the membrane easily and at a low cost. And thus the accomplishment of the present invention was arrived at.

In other words the present invention relates to a hydrophilized porous membrane for medical use, which comprises a hydrophobic porous membrane and a substance having surface activity, the substance being adsorbed on a surface of the hydrophobic porous membrane in an amount of 0.02 mg to 250 mg per dry weight (g) of the membrane, wherein the substance having surface activity is at least one member selected from polyoxyethylene sorbitan surfactant, purified vitelline lecithin, highly purified vitelline lecithin, purified soybean lecithin, and hydrogenated polyoxyethylene castor oil.

A preferable embodiment is a hydrophilized porous membrane in which the substance having surface activity has a number average molecular weight of 500 to 8,000.

A preferable embodiment is a hydrophilized porous membrane in which the polyoxyethylene sorbitan surfactant is at least one member selected from the group consisting of polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate.

A preferable embodiment is a hydrophilized porous membrane in which the hydrophobic porous membrane comprises polysulfone as the main structural component.

Furthermore, the present invention relates to a process for hydrophilization for medical equipment having a hydrophobic surface, which comprises impregnating a hydrophobic part to be in contact with blood into a solution of a substance having surface activity, thereby adsorbing the substance having surface activity on the hydrophobic surface in an amount of 0.02 mg to 250 mg per unit dry weight (g), wherein the substance having surface activity is at least one member selected from polyoxyethylene sorbitan surfactant, purified vitelline lecithin, highly purified vitelline lecithin, purified soybean lecithin, and hydrogenated polyoxyethylene castor oil.

A preferable embodiment is a process for hydrophilization in which the hydrophobic part to be in contact with blood is a porous membrane.

A preferable embodiment is a process for hydrophilization which further comprises after impregnating the membrane into a solution of a substance having surface activity, rinsing with a solvent in which the substance having surface activity is soluble, thereby eluting excess substance having surface activity.

Furthermore, the present invention relates to a process for hydrophilization for a hydrophobic porous membrane which comprises storing the membrane in a housing having at least a blood inflow part for blood to flow into the membrane and a blood outflow part for inflowing blood to flow out, passing a solution of a substance having surface activity through the housing and adsorbing the substance having surface activity on the surface of the membrane in an amount of 0.02 mg to 250 mg per unit dry weight (g) of the membrane within the housing, wherein the substance having surface activity is at least one member selected from polyoxyethylene sorbitan surfactant, purified vitelline lecithin, highly purified vitelline lecithin, purified soybean lecithin, and hydrogenated polyoxyethylene castor oil.

A preferable embodiment is a process for hydrophilization which further comprises after passing the solution of a substance having surface activity through the housing, rinsing with a solvent in which the substance having surface activity is soluble, thereby eluting excess substance having surface activity.

A preferable embodiment is a process for hydrophilization in which the substance having surface activity has a number average molecular weight of 500 to 8,000.

A preferable embodiment is a process for hydrophilization in which the polyoxyethylene sorbitan surfactant is at least one member selected from the group consisting of polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate.

A preferable embodiment is a process for hydrophilization in which the hydrophobic porous membrane comprises polysulfone as the main structural component.

In the present invention, a substance having surface activity is used as the hydrophilizing agent. Examples of the substance having surface activity are as defined above. Of these lecithin as defined and hydrogenated polyoxyethylene castor oil are usually used as a dispersant for intravenous injection preparation and are preferable as toxicity within the blood is particularly low. Examples of the polyoxyethylene sorbitan surfactant are polyoxyethylene sorbitan acyl ester, polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate. These may be used alone or in combination.

These surfactants preferably have a number average molecular weight of 500 to 8,000 from the viewpoint of preventing clogging of the membrane which accompanies hydrophilization of the membrane and rinsing efficiency. Also, a low toxic surfactant which is widely known to have little effect on the human body in the case that the surfactant eludes, is preferable. Polyoxyethylene sorbitan monooleate which is a polyoxyethylene sorbitan surfactant is preferable. Also, purified vitelline lecithin, highly purified vitelline lecithin and purified soybean lecithin which have been extensively used as intravenous injection preparation are preferable in that toxicity within blood is particularly low. In the same way, as a low toxic substance having surface activity, hydrogenated polyoxyethylene castor oil, hydrogenated polyoxyethylene castor oil 10, hydrogenated polyoxyethylene castor oil 40, hydrogenated polyoxyethylene castor oil 50 and hydrogenated polyoxyethylene castor oil 60 are also preferable and of these, hydrogenated polyoxyethylene castor oil 60 which has lower toxicity is more preferable. In the present invention, the hydrophilizing agent can also be a combination of at least two kinds.

When hydrophilizing, the solution into which the hydrophilizing agent is dissolved preferably comes into contact with the hydrophobic membrane at a temperature of 4°C to 70°C, more preferably, 4°C to 50°C from the viewpoint of production efficiency. The concentration of the hydrophilizing agent is preferably 0.001 % (W/W) to 10 % (W/W), more preferably 0.001 % (W/W) to 1 % (W/W) from the viewpoint of rinsing efficiency, further preferably 0.005 % (W/W) to 1 % (W/W) in order to obtain a sufficient effect of suppressing blood platelet adhesion.

The time of contact of the hydrophilizing agent and hydrophobic membrane can be a short period, impregnating the membrane into the hydrophilizing agent, but is preferably within 1 minute to 2 hours, in order to obtain a sufficient effect of suppressing blood platelet adhesion. More preferably, the time is within 2 minutes to 1.5 hours, from the viewpoint of adsorption stability of the hydrophilizing agent to the membrane and further preferably, the time is within 2 minute to 60 minutes, from the viewpoint of rinsing efficiency of the excess hydrophilizing agent. When the impregnation time is less than 1 minute, the adsorbed amount is insufficient and the effect of suppressing blood platelet adhesion tends to decrease. When the time is 2 hours or more, the adsorbed amount has reached equilibrium and so the effect of suppressing blood platelet adhesion will not change by further adsorption. Also, disadvantages in terms of production efficiency, such as the membrane rinsing time becoming longer, tend to be caused.

Example of the method of bringing the hydrophilizing agent and hydrophobic membrane into contact are the method of impregnating the membrane into the hydrophilizing agent, the method of impregnating and then shaking and the method comprising storing the membrane into a housing having an inflow part and an outflow part and then bringing the hydrophilizing agent and hydrophobic membrane into contact by passing the hydrophilizing agent through the housing.

After bringing the hydrophilizing agent and hydrophobic membrane into contact, the hydrophobic membrane is preferably rinsed with a solvent into which the hydrophilizng agent is soluble.

Example of the solvent which dissolves the hydrophilizing agent are water, aqueous solution containing electrolyte (saline, buffer solutions such as phosphate buffer solution), alcohols such as ethanol, warm ethanol and methanol, pyridine, chloroform, cyclohexane, ethyl acetate, toluene or a mixed solvent thereof. Water and an aqueous solution containing electrolyte are preferably used from the viewpoint of the effect to the material to be hydrophilized, after-treatment of the solvent, safety and cost.

By measuring the total organic carbon (TOC) concentration (JIS K 0551), whether rinsing was sufficiently conducted (for example, TOC value=0) can be confirmed.

The amount adsorbed of the hydrophilizing agent can be found indirectly by the above total organic carbon (TOC) method. More specifically, the amount adsorbed can be obtained by subtracting the amount of the hydrophilizing agent found by the TOC value of the hydrophilizing agent solution after hydrophilization and the TOC value of the rinsing solution of the membrane, from the amount of the hydrophilizing agent found by the TOC value of the solution dissolving the hydrophilizing agent before treatment. The nonionic surfactant can be directly quantified by an improved method (Miura et al, "Extracting a trace of nonionic surfactant using ammonium tetrathiocyanatocobaltate (II)/absorptiometry", (1989) Bunseki Kagaku) of the quantifying method using ammonium tetrathiocyanatocobaltate (II) (JIS K 3363).

The amount adsorbed of the hydrophilizing agent is 0.02 mg to 250 mg per unit dry weight (g) of the hydrophobic membrane. When the amount adsorbed of the hydrophilizing agent is at most 0.02 mg, the effect of suppressing blood platelet adhesion may not be sufficiently obtained. When the amount to be adsorbed is at least 250 mg, time and a great deal of rinsing solution becomes necessary for rinsing the membrane and rinsing tends to become inefficient. The amount is preferably 0.1 mg to 250 mg in order to suppress adhesion of blood cells, more preferably 0.1 mg to 125 mg from the viewpoint of suppressing elution of the hydrophilizing agent into the blood. At the same time, the amount is preferably 0.5 mg to 125 mg from the viewpoint of the effect of suppressing blood cell adhesion, more preferably 1.0 mg to 80 mg from the viewpoint of safety such as sufficient suppression of elution of the hydrophilizing agent and the effect of suppressing blood cell adhesion. Further preferably, the amount is 2.0 mg to 50 mg from the viewpoint of production efficiency such as shortening of hydrophilizing agent treatment time and reducing rinsing time after adsorbing the hydrophilizing agent and the amount to be rinsed.

The shape of the hydrophobic membrane used in the present invention is not particularly limited and may be a hollow fiber shape, tubular shape or a flat membrane. The material is preferably a polymer with relatively high hydrophobic properties which can adsorb an extremely small amount with stability, even after sufficiently rinsing the adsorbed hydrophilizing agent, such as polysulfone, polyethylene, polypropylene, polystyrene, polycarbonate, polyurethane and poly(methyl methacrylate). Of these, polysulfone is particularly preferable from the viewpoint of high adsorption stability of the hydrophilizing agent which is adsorbed in an extremely small amount, but in the present invention the material is not limited to these. The embodiment of the above hydrophobic membrane is preferably porous, in order to exhibit the effect of suppressing blood platelet adhesion by the hydrophilizing agent which is adsorbed in an extremely small amount.

The average pore size of the hydrophobic membrane is preferably 0.03 µm to 10 µm. When the pore size is smaller than 0.03 µm, rinsing takes a long time and the amount of unrinsed hydrophilizing agent tends to become large, and when the average pore size is greater than 10 µm, the strength tends to become weak due to the membrane structure.

When hollow fiber is used as the membrane and the membrane is porous, it is preferable that the average pore size is greater than 0.05 µm from the viewpoint of efficiency in rinsing the hydrophilizing agent and at most 4 µm in order to maintain sufficient membrane strength. Herein, the surface of the hydrophobic porous membrane signifies not only the area which comes into contact with blood but also all surfaces which may possibly come into contact with the hydrophilizing agent. Also, the adsorption state is not particularly limited, including homogenous adsorption to a monomolecular level, inhomogenous adsorption, localized adsorption and coagulated state adsorption. Examples of the method for confirming the adsorption state of the hydrophilizing agent are the method of confirming by a fluorescent microscope or a confocal laser scanning microscope after adsorbing fluorescent marked hydrophilizing agent or a direct method of confirming by a high resolution scanning electron microscope or atomic force microscope.

### EXAMPLE 1 (Reference example)

### [Preparation of the polysulfone sheet]

Approximately 20 % (W/V) of P-1700 Polysulfone (available from Teijin Amoco Co., Ltd) was put into N-methylpyrrolidone (NMP). The polysulfone was dissolved at 120°C and homogenous polysulfone dope was obtained.

After impregnating the glass tube in the above dope, the glass tube was slowly pulled out and impregnated in a coagulation bath (distilled water) to coagulate the polysulfone. The coagulated polysulfone was detached from the glass tube and cut into squares of a length of 4 × 4 mm to prepare the polysulfone sheet. The average pore size of the porous polysulfone membrane obtained here is 0.1 µm to 5 µm. Next, 40 ml of reverse osmosis water (hereinafter RO water) and the polysulfone sheet were put into a sample tube (100 ml) and after conducting heat treatment at 90°C for 30 minutes, decantation rinsing was conducted. After repeating this step a total of three times, the polysulfone sheet was further rinsed by repeating decantation rinsing by using 40 ml of RO water at room temperature five times.

### [Hydrophilization treatment]

120 sheets of the polysulfone sheet (4 × 4 mm) were put into a sample tube (30 ml). Thereto was added 15 ml of RO water into which 1 % (W/V) of polyoxyethylene cetyl ether (Brij 58, molecular weight 1,100) which is a polyoxyethylene alcohol ether nonionic surfactant was dissolved. Shaking was carried out at 20°C for 30 minutes at a rate of 100 times/minute to conduct hydrophilization.

After shaking, decantation rinsing was conducted 5 times by using 15 ml of RO water and after shaking further for 30 minutes in 15 ml of RO water, decantation rinsing was conducted. This step was repeated a total of three times.

Then, the RO water was removed by a pipet so that the polysulfone sheet would not be pulled, and 15 ml of new RO water was added and shaking was conducted for 30 minutes. The TOC of the supernatant was measured and this step was repeated to conduct rinsing of the hydrophilizing agent until the TOC value became 0 (Table 1). Finally, after conducting steam sterilization under high pressure at 121°C for 20 minutes, the TOC of the supernatant was measured (Table 1).

The TOC value of the polysulfone sheet after rinsing is 0 and indicates that rinsing was sufficient. Furthermore, the TOC value of the supernatant after conducting steam sterilization under high pressure is less than 5 ppm and indicates that elution of the hydrophilizing agent did not occur (Table 1).

Here, the amount of the polyoxyethylene cetyl ether adsorbed to the above membrane was 21 (mg/dry weight of the membrane g).

**TABLE 1**

| Elution of Brij 58 in Each Step (TOC Measurement) | | |
|---|---|---|
| Step | Type of solution used | Measurement result (ppm) |
| Rinsing | Distilled water | 0 |
| High pressure sterilization | Distilled water | Less than 5 |

### [Interaction with blood]

A total of 33 sheets of the polysulfone sheet were put into a PP tube (6 ml, Falcon 2063). Next, 5 ml of a saline containing heparin (heparin concentration 2 IU/ml) was added thereto and after agitating, the supernatant was removed. In the same way, a saline containing heparin was added and this step was conducted a total of 3 times for rinsing of the membrane. After sufficiently removing the supernatant, 1.5 ml of blood collected from a healthy volunteer (anticoagulated by 2 IU/ml of heparin) was added and reverse mixing was slowly conducted. Then, the tube was placed in a constant temperature water bath of 37°C and shaking was conducted for 40 minutes at a rate of 70 times/minute. After the specified time, 1 ml of the blood was added to a PP tube (6 ml, Falcon 2063) and the number of blood cells was counted by a blood cell counter (Mircocell Counter CC-180, made by Sysmex Corporation). The results are shown in Table 2.

### REFERENCE EXAMPLE 1

The interaction with blood was evaluated and the number of blood cells was found in the same manner as in Example 1, except that the polysulfone sheet was not inserted.

### COMPARATIVE EXAMPLE 1

The interaction with blood was evaluated in the same manner as in Example 1, except that a P-1700 polysulfone sheet to which hydrophilization was not conducted was used. The results are shown in Table 2.

### COMPARATIVE EXAMPLE 2

The interaction with blood was evaluated in the same manner as in Example 1, except that ethylene vinyl alcohol (EVAL, molecular weight approximately 5,000, available from Nippon Synthetic Chemical Industry Co., Ltd) was used as the typical hydrophilizing agent. The results are shown in Table 2.

### COMPARATIVE EXAMPLE 3

The interaction with blood was evaluated in the same manner as in Example 1, except that polyvinyl pyrrolidone (PVP, molecular weight approximately 8,000, available from Wako Pure Chemical Industries Ltd.) was used as the typical hydrophilizing agent. The results are shown in Table 2.

### EXAMPLE 2 (Reference example)

A polysulfone sheet (average pore size 0.09 µm to 4 µm) was prepared, hydrophilization was conducted by polyoxyethylene cetyl ether (Brij 58) which is a polyoxyethylene alcohol ether nonionic surfactant and the evaluation of interaction with blood and the measurement of the number of blood cells were conducted in the same manner as in Example 1, except that P-3500 polysulfone (available from Teijin Amoco Co., Ltd.) was used. The results are shown in Table 2.

Here, the amount of the polyoxyethylene cetyl ether adsorbed to the above membrane was 26 (mg/dry weight of the membrane g).

### COMPARATIVE EXAMPLE 4

The interaction with blood was evaluated in the same manner as in Example 1, except that a P-3500 polysulfone sheet to which hydrophilization was not conducted was used. The results are shown in Table 2.

### COMPARATIVE EXAMPLE 5

A hydrophilized sheet was prepared in the same manner as in Example 2, except that ethylene vinyl alcohol (EVAL) was used as the typical hydrophilizing agent and interaction with blood was evaluated in the same manner as in Example 1. The results are shown in Table 2.

### COMPARATIVE EXAMPLE 6

A hydrophilized sheet was prepared in the same manner as in Example 2, except that polyvinyl pyrrolidone (PVP) was used as the typical hydrophilizing agent and interaction with blood was evaluated in the same manner as in Example 1. The results are shown in Table 2.

The closer the number of blood cells counted is to the Reference Example, the lower the interaction between the blood cells and the polysulfone sheet. The smaller the number of the blood cells is the higher the interaction between the two and this signifies that adhesion and coagulation of the blood cells are occurring. As shown in Table 2, the fluctuation of the numbers of both erythrocytes and leukocytes remains within 10 % in the Examples and Comparative Examples, indicating that adhesion of erythrocytes and leukocytes has hardly occurred.

Furthermore, regarding blood platelets, the number of blood platelets in the case of using the polysulfone sheet prepared by using the hydrophilizing agent according to the hydrophilization method as described in Example 1 and 2 is closest to the value of Reference Example 1, indicating that adhesion of blood platelets has been greatly kept down (Table 2). On the other hand, in the Comparative Examples in which an unhydrophilized polysulfone sheet is used (Comparative Examples 1 and 4) and hydrophilization is conducted by an ordinary hydrophilizing agent (Comparative Examples 2, 3, 5 and 6), the decrease rate of the number of blood platelets is great, indicating that interaction with blood platelets is large (Table 2).

**TABLE 2**

| Interaction of hydrophilized membrane and blood cells | | | | | | | | | | | | | | | |
|---|---|---|---|---|---|---|---|---|---|---|---|---|---|---|---|
| | Number of blood cells | | | | | | | | | | | | | | |
| | Erythrocyte (× 10⁴ cells/µl) | | | | | Leukocyte (× 10² cells/µl) | | | | | Blood platelet (× 10⁴ cells/µl) | | | | |
| | n=1 | n=2 | n=3 | Average | ± SD | n=1 | n=2 | n=3 | Average | ±SD | n=1 | n=2 | n=3 | Average | ±SD |
| Ex. 1 | 469 | 565 | 465 | 500 | 57 | 59 | 70 | 34 | 54 | 18 | 18.0 | 12.7 | 13.3 | 14.7 | 2.9 |
| Ref. Ex. 1 | 501 | 555 | 494 | 517 | 33 | 69 | 86 | 47 | 67 | 20 | 18.5 | 18.8 | 15.1 | 17.5 | 2.1 |
| Com. Ex. 1 | 453 | 542 | 450 | 482 | 52 | 53 | 66 | 38 | 52 | 14 | 12.6 | 10.4 | 9.0 | 10.7 | 1.8 |
| Com. Ex. 2 | 447 | 577 | 454 | 493 | 73 | 54 | 68 | 34 | 52 | 17 | 15.1 | 12.3 | 10.0 | 12.5 | 2.6 |
| Com. Ex. 3 | 455 | 565 | 444 | 488 | 67 | 55 | 68 | 36 | 53 | 16 | 13.7 | 13.2 | 12.0 | 13.0 | 0.9 |
| Ex. 2 | 468 | 561 | 478 | 502 | 51 | 59 | 74 | 35 | 56 | 20 | 18.6 | 16.1 | 13.3 | 16.0 | 2.7 |
| Com. Ex. 4 | 449 | 569 | 460 | 493 | 66 | 60 | 72 | 38 | 57 | 17 | 14.6 | 14.1 | 11.1 | 13.3 | 1.9 |
| Com. Ex. 5 | 438 | 543 | 438 | 473 | 61 | 57 | 73 | 35 | 55 | 19 | 15.9 | 18.2 | 9.6 | 14.6 | 4.5 |
| Com. Ex. 6 | 453 | 588 | 466 | 502 | 74 | 56 | 77 | 36 | 56 | 21 | 15.1 | 13.9 | 11.7 | 13.6 | 1.7 |

### EXAMPLE 3

1 % (W/V) of polyoxyethylene sorbitan monooleate (Tween 80, available from Nikko Chemical Co., Ltd., molecular weight 1,611) which is a nonionic surfactant was dissolved into RO water and 1,000 ml of the solution was put into a triangular flask (temperature of the solution was 20°C). Next, all of the water (from both the QB side: side where blood flows in and out and QF side: plasma side which is the outside of the membrane) within the polysulfone plasma separation membrane (Sulflax-08, available from Kaneka Corporation) was extracted and the above Tween 80 solution was poured for 1 minute to the QB side by up flow at a flow rate of approximately 100 (ml/minute) using a roller pump. Then, the QB side is held by forceps and in the same way, the Tween 80 solution was poured for 1 minute to the QF side. The forceps on the QB side were removed and at QB=QF=50 (ml/minute), the above Tween 80 solution was further circulated for 5 minutes.

Rinsing was conducted by passing purified water which was passed through a 0.22 µm millipore filter (model number: MCGL 40503) (blowing after passing) through the QB side at 140 (ml/minute) and by blowing at 70 (ml/minute) to the QF side. Sampling of the blow liquid was conducted as time passed at both the QB side and QF side and the TOC was measured. Rinsing was conducted until the TOC value reached 0 (Table 3).

The plasma separation membrane after rinsing was subjected to γ-ray sterilization (50 KGy) and after the filling liquid was sterilized, the membrane was sampled for testing elution and measurement was carried out by a quantification method improved from the quantification method using ammonium tetrathiocyanatocobaltate (II) (JIS K3363) (Table 3). Here, the amount of Tween 80 adsorbed to the membrane was 27 (mg/dry weight of the membrane g).

For extracting Tween 80 after γ-ray sterilization, 500 ml of saline per 2 of the above membrane was used and circulation was conducted for 2 hours at a temperature of 40°C at 130 ml/minute for the QB side and 30 ml/minute for the QF side. After concentrating by using an evaporator, the extract liquid was measured by the quantification method improved from the quantification method using ammonium tetrathiocyanatocobaltate (II) (JIS K3363) (Table 3).

**TABLE 3**

| Elution of Tween 80 in Each Step (TOC Measurement) | | |
|---|---|---|
| Step | Type of solution used | Measurement result |
| Rinsing | Distilled water | 0 (ppm)¹⁾ |
| γ-ray sterilization | Distilled water | Less than 10 (mg/l) ²⁾ |
| Extraction (after sterilization) | Saline | Less than 10 (mg/l) ²⁾ |

| | | |
|---|---|---|
| ¹⁾ TOC measurement ²⁾ Improved method of JIS K3363 | | |

### [Interaction with blood]

A membrane is cut out from the inside of the plasma separation membrane and 50 strips of the membrane cut into a length of 1 cm were prepared. Furthermore, the membrane was cut into 2 segments (a total of 100 strips) in the length direction (vertically) so that the blood can sufficiently come into contact with the inner surface of the membrane. The membrane was put into a PP tube (6 ml, Falcon 2063). Next, 5 ml of a saline containing heparin (heparin concentration 2 IU/ml) was added thereto and after agitating, the supernatant was removed. In the same way, a saline containing heparin was added again and this step was conducted a total of 3 times for rinsing of the membrane. After sufficiently removing the supernatant, 1.5 ml of blood collected from a healthy volunteer (anticoagulated by 2 IU/ml of heparin) was added and reverse mixing was slowly conducted. Then, the tube was placed in a constant temperature water bath of 37°C and shaking was conducted for 40 minutes at a rate of 70 times/minute. After the specified time, 1 ml of the blood was added to a PP tube (6 ml, Falcon 2063) and the number of blood cells was counted by a blood cell counter (Mircocell Counter CC-180, made by Sysmex Corporation).

### EXAMPLE 4

Treatment and evaluation were conducted in the same way as in Example 3 except that polyoxyethylene sorbitan monolaurate (Tween 20, available from Nikko Chemical Co., Ltd., molecular weight 1,000) which is a nonionic surfactant was used and the results are shown in Table 4.

Here, the amount of the polyoxyethylene sorbitan monolaurate adsorbed to the above membrane was 19 (mg/dry weight of the membrane g).

### EXAMPLE 5

Treatment and evaluation were conducted in the same way as in Example 3 except that purified vitelline lecithin (available from Wako Pure Chemical Industries Ltd., molecular weight 1,000) was used as the hydrophilizing agent and the results are shown in Table 4.

Here, the amount of the purified vitelline lecithin adsorbed to the above membrane was 33 (mg/dry weight of the membrane g).

### EXAMPLE 6

Treatment and evaluation were conducted in the same way as in Example 3 except that hydrogenated polyoxyethylene castor oil 60 (available from Wako Pure Chemical Industries Ltd., molecular weight 600) was used as the hydrophilizing agent and the results are shown in Table 4.

Here, the amount of the hydrogenated polyoxyethylene castor oil 60 adsorbed to the above membrane was 48 (mg/dry weight of the membrane g).

### REFERENCE EXAMPLE 2

Treatment and evaluation were conducted in the same way as in Example 3 except that the polysulfone membrane was not used and the results are shown in Table 4.

### COMPARATIVE EXAMPLE 7

Treatment and evaluation were conducted in the same way as in Example 3 except that the polysulfone membrane used in Example 3 was used without conducting hydrophilization and the results are shown in Table 4.

### COMPARATIVE EXAMPLE 8

Treatment and evaluation were conducted in the same way as in Example 3 except that the polyethylene glycol (available from Wako Pure Chemical Industries Ltd., molecular weight 8,000) was used and the results are shown in Table 4.

### COMPARATIVE EXAMPLE 9

Treatment and evaluation were conducted in the same way as in Example 3 except that the polypropylene glycol (available from Asahi Glass Co., Ltd., molecular weight 4,000) was used and the results are shown in Table 4.

The adhesion rate of erythrocytes is low in both Examples and Comparative Examples and is less than 10 %, indicating that hardly any adhered (Table 4).

The adhesion rate of leukocytes is approximately 15 to 30 % in Comparative Examples, while in Examples, the rate remains approximately 10 to 15 % and so the effect of suppressing adhesion of leukocytes has been recognized (Table 4).

The adhesion rate of blood platelets is less than 5 % in Examples 3, 4, 5 and 6, indicating that the adhesion of blood platelets to the membrane has been significantly suppressed (Table 4). On the other hand, in Comparative Examples in which an unhydrophilized polysulfone membrane is used (Comparative Example 7) and hydrophilization is conducted by an ordinary hydrophilizing agent (Comparative Examples 8 and 9), the number of blood platelets decreases significantly, indicating that adhesion to the membrane is great (Table 4).

**TABLE 4**

| Interaction of hydrophilized membrane and blood cells | | | | | | | | | | | | | | | |
|---|---|---|---|---|---|---|---|---|---|---|---|---|---|---|---|
| | Number of blood cells | | | | | | | | | | | | | | |
| | Erythrocyte (× 10⁴ cells/µl) | | | | | Leukocyte (× 10² cells/µl) | | | | | Blood platelet (× 10⁴ cells/µl) | | | | |
| | n=1 | n=2 | n=3 | Average | ± SD | n=1 | n=2 | n=3 | Average | ± SD | n=1 | n=2 | n=3 | Average | ± SD |
| Ex. 3 | 472 | 540 | 518 | 510 | 35 | 40 | 58 | 35 | 44 | 12 | 11.6 | 14.7 | 11.1 | 12.5 | 2.0 |
| Ex. 4 | 471 | 539 | 549 | 520 | 42 | 43 | 62 | 38 | 48 | 13 | 11.9 | 19.1 | 13.4 | 14.8 | 3.8 |
| Ex. 5 | 480 | 545 | 548 | 524 | 38 | 42 | 63 | 37 | 47 | 14 | 10.1 | 16.2 | 9.7 | 12.0 | 3.6 |
| Ex.6 | 496 | 515 | 548 | 520 | 26 | 37 | 61 | 40 | 46 | 13 | 12.4 | 17.4 | 13.2 | 14.3 | 2.7 |
| Ref. Ex. 2 | 504 | 576 | 556 | 545 | 37 | 44 | 76 | 41 | 54 | 19 | 11.1 | 17.3 | 13.9 | 14.1 | 3.1 |
| Com. Ex. 7 | 479 | 559 | 533 | 524 | 41 | 36 | 58 | 38 | 44 | 12 | 6.3 | 9.7 | 6.5 | 7.5 | 1.9 |
| Com. Ex. 8 | 484 | 538 | 490 | 504 | 30 | 36 | 42 | 31 | 36 | 6 | 9.7 | 11.7 | 8.4 | 9.9 | 1.7 |
| Com. Ex. 9 | 489 | 544 | 533 | 522 | 29 | 39 | 51 | 34 | 41 | 9 | 8.6 | 11.5 | 8 | 9.4 | 1.9 |

From all of the above, exhibiting a high effect of suppressing blood cell adhesion has become possible, even after autoclave or γ-ray sterilization, by an extremely easy and low cost method of adsorbing a substance having surface activity with a number average molecular weight of 500 to 8,000 and then sufficiently rinsing so that actually only an extremely small amount is adsorbed to the membrane.

According to the present invention, easy and low cost hydrophilization which does not cause deterioration or decrease in strength of the membrane became possible and adhesion of leukocytes and blood platelets was greatly suppressed. As a result, stable blood flow has become available, for example in medical equipment which was limited in use due to problems such as blood cell adhesion. Furthermore, after hydrophilization, the membrane can be stored in a dry state or in contact with a saline or distilled water and can maintain high compatibility with blood over a long period even after sterilization by an autoclave or γ-ray irradiation.

## Claims

1. A hydrophilized porous membrane for medical use, which comprises:
a hydrophobic porous membrane, and
a substance having surface activity, said substance being adsorbed on a surface of the hydrophobic porous membrane in an amount of 0.02 mg to 250 mg per dry weight (g) of said membrane,
wherein the substance having surface activity is at least one member selected from a polyoxyetylene sorbitan surfactant, purified vitelline lecithin, highly purified vitelline lecithin, purified soybean lecithin, and hydrogenated polyoxyethylene castor oil.

2. The hydrophilized porous membrane of Claim 1, wherein said substance having surface activity has a number average molecular weight of 500 to 8,000.

3. The hydrophilized porous membrane of Claim 1 or 2, wherein said polyoxyethylene sorbitan surfactant is at least one member selected from the group consisting of polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate.

4. The hydrophilized porous membrane of Claim 1, 2, or 3, wherein said hydrophobic porous membrane comprises polysulfone as the main structural component.

5. A process for hydrophilization for medical equipment having a hydrophobic surface, which comprises:
impregnating a hydrophobic part to be in contact with blood into a solution of a substance having surface activity, thereby adsorbing said substance having surface activity on said hydrophobic surface in an amount of 0.02 mg to 250 mg per unit dry weight (g), wherein the substance having surface activity is at least one member selected from a polyoxyethylene sorbitan surfactant, purified vitelline lecithin, highly purified vitelline lecithin, purified soybean lecithin, and hydrogenated polyoxyethylene castor oil.

6. The process for hydrophilization of Claim 5, wherein said hydrophobic part to be in contact with blood is a porous membrane.

7. The process for hydrophilization of Claim 5, which further comprises after impregnating into a solution of a substance having surface activity, rinsing with a solvent in which said substance having surface activity is soluble, thereby eluting excess substance having surface activity.

8. A process for hydrophilization for a hydrophobic porous membrane, which comprises:
storing said membrane in a housing having at least a blood inflow part for blood to flow into said membrane and a blood outflow part for inflowing blood to flow out;
passing a solution of a substance having surface activity through said housing and
adsorbing said substance having surface activity on a surface of said membrane in an amount of 0.02 mg to 250 mg per unit dry weight (g) of said membrane within said housing, wherein the substance having surface activity is at least one member selected from a polyoxyethylene sorbitan surfactant, purified vitelline lecithin, highly purified vitelline lecithin, purified soybean lecithin, and hydrogenated polyoxyethylene castor oil.

9. The process for hydrophilization of Claim 8, which further comprises after passing a solution of a substance having surface activity through said housing, rinsing with a solvent in which said substance having surface activity is soluble, thereby eluting excess substance having surface activity.

10. The process for hydrophilization of Claim 5, 6, 7, 8 or 9, wherein said substance having surface activity has a number average molecular weight of 500 to 8,000.

11. The process for hydrophilization of Claim 5, 6, 7, 8, 9 or 10, wherein said polyoxyethylene sorbitan surfactant is at least one member selected from the group consisting of polyoxyethylene sorbitan monolaurate, polyoxyethylene sorbitan monopalmitate, polyoxyethylene sorbitan monostearate, polyoxyethylene sorbitan tristearate and polyoxyethylene sorbitan monooleate.

12. The process for hydrophilization of Claim 5, 6, 7, 8, 9, 10 or 11,
wherein said hydrophobic porous membrane comprises polysulfone as the main structural component.

## Patentansprüche

1. Hydrophilisierte poröse Membran zur medizinischen Verwendung, umfassend:
eine hydrophobe poröse Membran und
eine Substanz mit Oberflächenaktivität, wobei die Substanz auf einer Oberfläche der hydrophoben porösen Membran in einer Menge von 0,02 mg bis 250 mg, bezogen auf das Trockengewicht (g) der Membran, adsorbiert ist, wobei die Substanz mit Oberflächenaktivität mindestens ein aus einem grenzflächenaktiven Mittel aus Polyoxyethylensorbitan, gereinigtem Vitellinlecithin, hochgereinigtem Vitellinlecithin, gereinigtem Sojabohnenlecithin und hydriertem Polyoxyethylenrizinusöl ausgewählter Bestandteil ist.

2. Hydrophilisierte poröse Membran gemäß Anspruch 1, wobei die Substanz mit Oberflächenaktivität ein Zahlenmittel des Molekulargewichts von 500 bis 8000 aufweist.

3. Hydrophilisierte poröse Membran gemäß Anspruch 1 oder 2, wobei das grenzflächenaktive Mittel aus Polyoxyethylensorbitan mindestens ein aus Polyoxyethylensorbitanmonolaurat, Polyoxyethylensorbitanmonopalmitat, Polyoxyethylensorbitanmonostearat, Polyoxyethylensorbitantristearat und Polyoxyethylensorbitanmonooleat ausgewählter Bestandteil ist.

4. Hydrophilisierte poröse Membran gemäß Anspruch 1, 2 oder 3, wobei die hydrophobe poröse Membran Polysulfon als Hauptstrukturkomponente umfasst.

5. Verfahren zum Hydrophilisieren von medizinischer Ausrüstung mit hydrophober Oberfläche, welches umfasst:
Imprägnieren eines zum Kontakt mit Blut vorgesehenen hydrophoben Teils in einer Lösung einer Substanz mit Oberflächenaktivität, wodurch die Substanz mit Oberflächenaktivität auf der hydrophoben Oberfläche in einer Menge von 0,02 mg bis 250 mg, bezogen auf die Trockengewichtseinheit (g), adsorbiert wird, wobei die Substanz mit Oberflächenaktivität mindestens ein aus einem grenzflächenaktiven Mittel aus Polyoxyethylensorbitan, gereinigtem Vitellinlecithin, hochgereinigtem Vitellinlecithin, gereinigtem Sojabohnenlecithin und hydriertem Polyoxyethylenrizinusöl ausgewählter Bestandteil ist.

6. Verfahren zum Hydrophilisieren gemäß Anspruch 5, wobei der zum Kontakt mit Blut vorgesehene hydrophobe Teil eine poröse Membran ist.

7. Verfahren zum Hydrophilisieren gemäß Anspruch 5, weiterhin umfassend nach dem Imprägnieren in einer Lösung einer Substanz mit Oberflächenaktivität, Spülen mit einem Lösungsmittel, in welchem die Substanz mit Oberflächenaktivität löslich ist, wodurch überschüssige Substanz mit Oberflächenaktivität eluiert wird.

8. Verfahren zum Hydrophilisieren für eine hydrophobe poröse Membran, welches umfasst:
Lagern der Membran in einem Gehäuse mit mindestens einem Bluteinlassteil für in die Membran fließendes Blut und einem Blutauslassteil zum Ausfließen des einfließenden Blutes;
Durchleiten einer Lösung einer Substanz mit Oberflächenaktivität durch das Gehäuse und Adsorbieren der Substanz mit Oberflächenaktivität auf einer Oberfläche der Membran in einer Menge von 0,02 mg bis 250 mg, bezogen auf die Trockengewichtseinheit (g) der Membran, innerhalb des Gehäuses, wobei die Substanz mit Oberflächenaktivität mindestens ein aus einem grenzflächenaktiven Mittel aus Polyoxyethylensorbitan, gereinigtem Vitellinlecithin, hochgereinigtem Vitellinlecithin, gereinigtem Sojabohnenlecithin und hydriertem Polyoxyethylenrizinusöl ausgewählter Bestandteil ist.

9. Verfahren zum Hydrophilisieren gemäß Anspruch 8, weiterhin umfassend nach dem Durchleiten einer Lösung einer Substanz mit Oberflächenaktivität durch das Gehäuse, Spülen mit einem Lösungsmittel, in welchem die Substanz mit Oberflächenaktivität löslich ist, wodurch überschüssige Substanz mit Oberflächenaktivität eluiert wird.

10. Verfahren zum Hydrophilisieren gemäß Anspruch 5, 6, 7, 8 oder 9, wobei die Substanz mit Oberflächenaktivität ein Zahlenmittel des Molekulargewichts von 500 bis 8000 aufweist.

11. Verfahren zum Hydrophilisieren gemäß Anspruch 5, 6, 7, 8, 9 oder 10, wobei das grenzflächenaktive Mittel aus Polyoxyethylensorbitan mindestens ein aus Polyoxyethylensorbitanmonolaurat, Polyoxyethylensorbitanmonopalmitat, Polyoxyethylensorbitanmonostearat, Polyoxyethylensorbitantristearat und Polyoxyethylensorbitanmonooleat ausgewählter Bestandteil ist.

12. Verfahren zum Hydrophilisieren gemäß Anspruch 5, 6, 7, 8, 9, 10 oder 11, wobei die hydrophobe poröse Membran Polysulfon als Hauptstrukturkomponente umfasst.

## Revendications

1. Membrane poreuse hydrophilisée à usage médical, qui comprend :
une membrane poreuse hydrophobe, et
une substance ayant une activité de surface, ladite substance étant adsorbée sur une surface de la membrane poreuse hydrophobe en une quantité allant de 0,02 mg à 250 mg basée
sur le poids sec (g) de ladite membrane, dans laquelle la substance ayant une activité de surface est au moins un élément choisi parmi un agent tensio-actif à base de polyoxyéthylène sorbitane, de lécithine vitelline purifiée, de lécithine vitelline hautement purifiée, de lécithine de soja purifiée et de polyoxyéthylène huile de ricin hydrogénée.

2. Membrane poreuse hydrophilisée selon la revendication 1, dans laquelle ladite substance ayant une activité de surface a une masse moléculaire moyenne en nombre dans la plage de 500 à 8 000.

3. Membrane poreuse hydrophilisée selon la revendication 1 ou la revendication 2, dans laquelle ledit agent tensio-actif à base de polyoxyéthylène sorbitane est au moins un élément choisi dans le groupe constitué par le monolaurate de polyoxyéthylène sorbitane, le monopalmitate de polyoxyéthylène sorbitane, le monostéarate de polyoxyéthylène sorbitane, le tristéarate de polyoxyéthylène sorbitane et le monooléate de polyoxyéthylène sorbitane.

4. Membrane poreuse hydrophilisée selon la revendication 1, 2 ou 3, dans laquelle ladite membrane poreuse hydrophobe comprend une polysulfone en tant que composant structurel principal.

5. Procédé d'hydrophilisation d'un équipement médical ayant une surface hydrophobe, qui comprend :
l'imprégnation d'une partie hydrophobe destinée à être en contact avec du sang dans une solution d'une substance ayant une activité de surface, afin d'adsorber ainsi ladite substance ayant une activité de surface sur ladite surface hydrophobe en une quantité allant de 0,02 mg à 250 mg par unité de poids sec (g), dans lequel la substance ayant une activité de surface est au moins un élément choisi parmi un agent tensio-actif à base de polyoxyéthylène sorbitane, de lécithine vitelline purifiée, de lécithine vitelline hautement purifiée, de lécithine de soja purifiée et de polyoxyéthylène huile de ricin hydrogénée.

6. Procédé d'hydrophilisation selon la revendication 5, dans lequel ladite partie hydrophobe destinée à être en contact avec du sang est une membrane poreuse.

7. Procédé d'hydrophilisation selon la revendication 5, qui comprend en plus, après l'imprégnation dans une solution d'une substance ayant une activité de surface, le rinçage avec un solvant dans lequel ladite substance ayant une activité de surface est soluble afin d'éluer ainsi l'excès de substance ayant une activité de surface.

8. Procédé d'hydrophilisation d'une membrane poreuse hydrophobe, qui comprend les étapes consistant à :
stocker ladite membrane dans un boîtier ayant au moins une partie d'entrée de sang permettant à du sang de pénétrer dans ladite membrane et une partie de sortie de sang permettant au sang entrant de s'écouler hors de la membrane ;
faire passer une solution d'une substance ayant une activité de surface dans ledit boîtier ; et
adsorber ladite substance ayant une activité de surface sur une surface de ladite membrane en une quantité allant de 0,02 mg à 250 mg par unité de poids sec (g) de ladite membrane dans ledit boîtier, dans lequel la substance ayant une activité de surface est au moins un élément choisi parmi un agent tensio-actif à base de polyoxyéthylène sorbitane, de lécithine vitelline purifiée, de lécithine vitelline hautement purifiée, de lécithine de soja purifiée et de polyoxyéthylène huile de ricin hydrogénée.

9. Procédé d'hydrophilisation selon la revendication 8, qui comprend en plus, après le passage d'une solution d'une substance ayant une activité de surface dans ledit boîtier, le rinçage avec un solvant dans lequel ladite substance ayant une activité de surface est soluble afin d'éluer ainsi l'excès de substance ayant une activité de surface.

10. Procédé d'hydrophilisation selon la revendication 5, 6, 7, 8 ou 9, dans lequel ladite substance ayant une activité de surface a une masse moléculaire moyenne en nombre dans la plage de 500 à 8 000.

11. Procédé d'hydrophilisation selon la revendication 5, 6, 7, 8, 9 ou 10, dans lequel ledit agent tensio-actif à base de polyoxyéthylène sorbitane est au moins un élément choisi dans le groupe constitué par le monolaurate de polyoxyéthylène sorbitane, le monopalmitate de polyoxyéthylène sorbitane, le monostéarate de polyoxyéthylène sorbitane, le tristéarate de polyoxyéthylène sorbitane et le monooléate de polyoxyéthylène sorbitane.

12. Procédé d'hydrophilisation selon la revendication 5, 6, 7, 8, 9, 10 ou 11, dans lequel ladite membrane poreuse hydrophobe comprend une polysulfone en tant que composant structurel principal.
